# EUROPEAN PATENT SPECIFICATION

(11) **EP 3 366 246 B1**
(45) Date of publication and mention of the grant of the patent: **28.06.2023**
(21) Application number: 16856948.1
(22) Date of filing: 22.10.2016
(51) Int. Cl.: A61B 18/14, A61B 18/00

(54) **RADIO FREQUENCY ABLATION DEVICE COMPRISING BALLOON BLOCKING CATHETER AND ABLATION METHOD THEREFOR**
FUNKFREQUENZABLATIONSVORRICHTUNG MIT BALLONBLOCKIERUNGSKATHETER UND ABLATIONSVERFAHREN DAFÜR
DISPOSITIF D'ABLATION PAR RADIOFRÉQUENCE COMPRENANT UN CATHÉTER À BALLONNET BLOQUANT ET PROCÉDÉ D'ABLATION ASSOCIÉ

(30) Priority: 22.10.2015 CN 201510689867; 22.10.2015 CN 201520821938 U
(43) Date of publication of application: 29.08.2018
(73) Proprietor: Shanghai Golden Leaf Med Tec Co., Ltd., Shanghai 200231 (CN)
(72) Inventor: DONG, Yonghua, Shanghai 200231 (CN); SHI, Zhengmin, Shanghai 200231 (CN); SHEN, Meijun, Shanghai 200231 (CN); GUO, Jiulin, Shanghai 200231 (CN)
(74) Representative: Petraz, Gilberto Luigi
(86) International application number: PCT/CN2016/102980
(87) International publication number: WO 2017/067517

(56) References cited:
- WO-A1-01/97897
- WO-A2-02/056772
- CN-A- 103 917 184
- CN-A- 103 987 336
- CN-A- 105 147 389
- CN-U- 203 524 766
- CN-U- 205 163 235
- US-A1- 2004 162 555
- US-A1- 2009 099 560

## Description

### BACKGROUND

### Technical Field

The present invention relates to a radio frequency ablation device, and in particular, to a radio frequency ablation device comprising a balloon blocking guide catheter, and belongs to the field of nerve ablation technologies.

### Related Art

Abnormalities of a vegetative nerve play a very important role in occurrence, evolution, and development of many diseases. Recently, with the development of minimally invasive techniques, the nerve ablation technique gradually has been applied to the clinic to give treatments for symptoms such as hypertension, diabetes, heart diseases, and cancerous tumors, and has achieved good effects.

A radio frequency ablation catheter is an important device for conducting radio frequency ablation. A guide catheter is a tube disposed outside the radio frequency ablation catheter. The radio frequency ablation catheter usually needs assistance of the guide catheter to establish a path from the in vitro to the heart or the renal artery. During the ablation operation, most parts of the ablation catheter is kept inside the guide catheter.

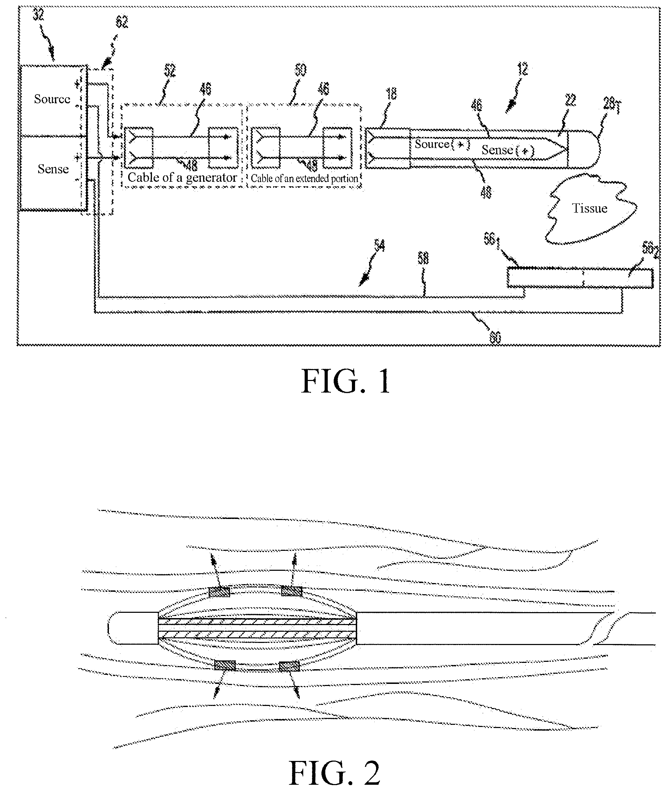

In the existing technology, the radio frequency ablation device includes a radio frequency ablation instrument provided with a radio frequency generator, a radio frequency ablation catheter, and a radio frequency electrode that connected to an output terminal of the radio frequency generator by using a wire disposed inside the radio frequency ablation catheter, and further includes a loop electrode connected to a loop terminal of the radio frequency generator. In the existing nerve ablation operation for a peripheral nerve of a lumen, the loop electrode of the radio frequency ablation device is usually disposed as a peripheral patch electrode, and is disposed in vitro. For a specific structure of the radio frequency ablation device, refer to the Chinese patent (Patent application number: CN200880126859.X) entitled "SYSTEM AND METHOD FOR MEASUREMENT OF IMPEDANCE USING CATHETER SUCH AS ABLATION CATHETER". As shown in FIG. 1, the patent discloses a catheter and a patch electrode system. A positive electrode of a radio frequency ablation generator 16 is electrically coupled to a point electrode 28T by using a source lead 46, a positive electrode of a sensing connector 32 is electrically coupled to the point electrode 28T by using a sensing lead 48, and a source loop 56₁ and a sensing loop 56₂ are respectively electrically connected to a negative electrode of the radio frequency ablation generator 16 and a negative electrode of the sensing connector. The source loop 56₁ and the sensing loop 56₂ are disposed in vitro. An excitation signal emitted by the point electrode 28T disposed inside the lumen needs to pass through the entire human tissue in vitro, and arrives at the radio frequency ablation generator 16 through the source loop 56₁. To be specific, a radio frequency current needs to enters the human tissue through walls in the lumen such as blood vessels, and returns to the radio frequency ablation generator from the in vitro after passing through the entire human body loop. In short, the radio frequency current needs to pass through the entire human tissue. During the radio frequency ablation, a radio frequency direction of the radio frequency electrode is shown in FIG. 2. In the nerve ablation manner in which a radio frequency loop includes the human body, large human body impedance needs to be overcome. Therefore, large voltage, current, and radio frequency power need to be used, which inevitably injures the blood vessels of the human body.

In addition, in the existing technology, there is a device in which a radio frequency electrode and a loop electrode are disposed on a same support to ablate pathological tissues, for example, a scissor radio frequency ablation device provided in the Chinese patent (Patent application number: CN201210128849.8) entitled "MULTIFUNCTIONAL RADIO FREQUENCY COOLING KNIFE". As shown in FIG. 8, the multifunctional radio frequency cooling knife includes a first branch 10, a second branch 20, a connection end portion 30, and an operation handle 40. Two bipolar radio frequency electrodes are disposed on each of the first branch 10 and the second branch 20. A radio frequency signal between two bipolar radio frequency electrodes disposed on each branch enters inside the tissue from the outside of the tissue through the human tissue, and returns to a local radio frequency loop outside the tissue for radio frequency ablation. In the radio frequency ablation device, the radio frequency electrode and the loop electrode are both disposed inside the human body but outside the to-be-ablated tissue. During the ablation process, no flowing blood affecting the radio frequency loop exists near the to-be-ablated tissue. When the ablation catheter in which the radio directly placed inside the lumen to ablate a nerve plexus near the vessel, because flowing blood exists inside the lumen, conductivity of the blood affects formation of the radio frequency loop. As a result, it is not easy to form a loop between two electrodes and passing through the blood vessel wall or the tissue outside the vessel, and in this case, it is difficult to achieve the ideal effect during ablation of the nerve near the lumen.

US 2004/162555A1 discloses a catheter includes a plurality of primary leads to deliver energy for ligating a hollow anatomical structure. Each of the primary leads includes an electrode located at the working end of the catheter. Separation is maintained between the primary leads such that each primary lead can individually receive power of selected polarity. The primary leads are constructed to expand outwardly to place the electrodes into apposition with a hollow anatomical structure. High frequency energy can be applied from the leads to create a heating effect in the surrounding tissue of the anatomical structure. The diameter of the hollow anatomical structure is reduced by the heating effect, and the electrodes of the primary leads are moved closer to one another. Where the hollow anatomical structure is a vein, energy is applied until the diameter of the vein is reduced to the point where the vein is occluded.

### SUMMARY

The primary technical problem to be resolved in the present invention is to provide a radio frequency ablation device comprising a balloon blocking guide catheter.

To achieve the foregoing invention objective, the following technical solutions are used in the present invention.

A radio frequency ablation device according to claim 1 is provided. The scope of the invention is defined by the independent claim. Preferred embodiments are defined in the dependent claims.

### BRIEF DESCRIPTION OF THE DRAWINGS

FIG. 1 is a schematic structural diagram of a radio frequency ablation system using a peripheral patch electrode as a loop electrode in the existing technology;
FIG. 2 is shows a radio frequency direction when a radio frequency is released to a lumen in the radio frequency ablation system shown in FIG. 1;
FIG. 3 is a schematic structural diagram of a radio frequency cooling knife used to cut off or ablate a pathological tissue from the outside of the pathological tissue in another existing technology;
FIG. 4 is a schematic diagram of a basic structure of a balloon blocking guide catheter according to the present invention;
FIG. 5 is a schematic diagram of a working state when a radio frequency ablation catheter passes through the balloon blocking guide catheter shown in FIG. 4;
FIG. 6 is a principle diagram of radio frequency ablation after the radio frequency ablation device provided in the present invention is used and a non-conductive liquid is infused into a lumen of a to-be-ablated portion;
FIG. 7 is a schematic diagram of the radio frequency ablation principle shown in FIG. 6 on a cross section of a blood vessel; and
FIG. 8 is a principle diagram of radio frequency ablation after the radio frequency ablation device provided in the present invention is used and a conductive liquid is infused into a lumen of a to-be-ablated portion.

### DETAILED DESCRIPTION

The following gives a detailed description with reference to the accompanying drawings and specific embodiments. For the convenience of description, an end close to a manipulator (away from a to-be-ablated portion) is referred to as a near end, and an end away from the manipulator (close to the to-be-ablated portion) is referred to as a far end.

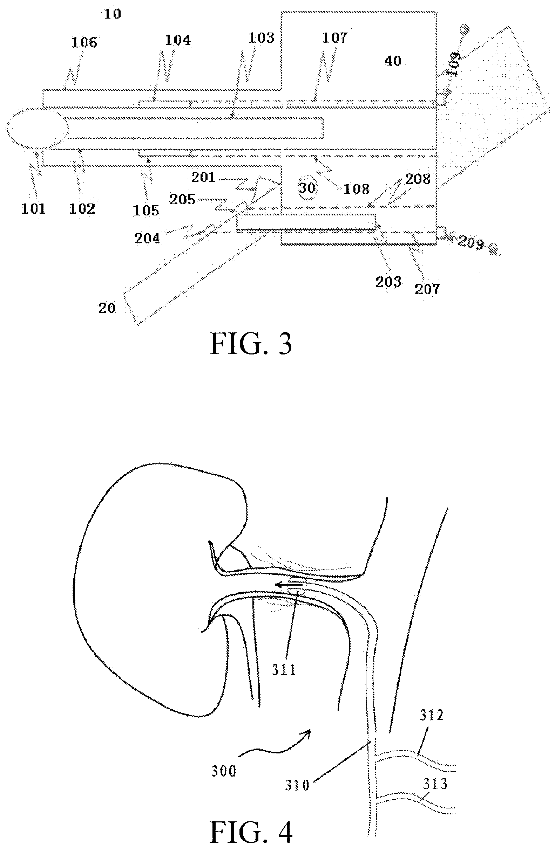

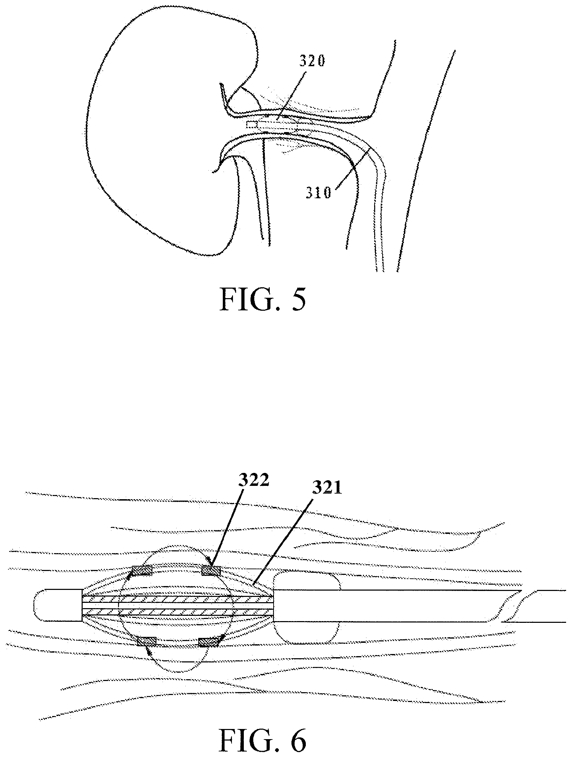

To change an ablation mode in the existing technology that when a peripheral nerve of a lumen is ablated, a peripheral patch electrode needs to be disposed in vitro as a loop electrode, and the peripheral patch electrode and the radio frequency electrode form a human body loop to conduct radio frequency ablation. The present invention provides a radio frequency ablation device 300 shown in FIG. 4 and FIG. 5 and comprising a balloon blocking guide catheter, a radio frequency ablation catheter, and a radio frequency generator (not shown in the figure), and further provides a radio frequency ablation method for ablating a peripheral nerve of a lumen by using the radio frequency ablation device. The radio frequency ablation device and the radio frequency ablation method may be used to give a radio frequency treatment to a cavity tissue, for example, ablation treatment to a vein, an endometrium, and an esophagus.

As shown in FIG. 4, the balloon blocking guide catheter 310 is a dual-chamber or multi-chamber catheter. An inflatable closed balloon 311 is disposed on an outer wall of a far end (namely, an end entering inside the human body) of a guide catheter 310. A first catheter branch 312 communicating with a first lumen inside the guide catheter is disposed on a near end (namely, an end away from the human body) of the guide catheter, the inside of the closed balloon 311 communicates with the first lumen inside the guide catheter 310, and the first catheter branch 312 is used to provide a inflation material for the closed balloon 311 through the first lumen. Specifically, an inflation device (not shown in the figure) connected to a tail end of the first catheter branch 312 infuses gas or liquid into the closed balloon 311, to implement an inflation effect of the closed balloon 311. A second catheter branch 313 communicating with a second lumen (namely, an infusion lumen) inside the guide catheter 310 is further disposed on the near end of the guide catheter 310. An infusion port is disposed on a far end of the infusion lumen, and the infusion lumen is used to infuse liquid/gas into a blood vessel on a to-be-ablated portion through the infusion port. An infusion device (not shown in the figure) is connected to a tail end of the second catheter branch 313, and is used to infuse liquid, for example, a contrast agent, into the blood vessel on the to-be-ablated portion through the second catheter branch 313, the infusion lumen, and the infusion port. For an infusion direction, refer to an arrow inside the blood vessel in FIG. 4. In addition, gas, for example, CO₂, may be alternatively infused into the blood vessel on the to-be-ablated portion. The closed balloon 311 may be aligned with the infusion port on the far end of the guide catheter 1, or a distance may exist between the closed balloon 311 and the infusion port on the far end of the guide catheter 1, to help blocking flowing blood inside the blood vessel and infuse liquid/gas into the blood vessel. In the balloon blocking guide catheter 310, local blood flow inside the blood vessel on the to-be-ablated portion can be blocked by inflating the closed balloon 311 on the far end, and a temperature environment or a conducting environment inside the blood vessel on the to-be-ablated portion can be changed by infusing liquid (for example, non-conductive liquid) into the blood vessel on the to-be-ablated portion.

As shown in FIG. 5, the radio frequency ablation catheter 320 is disposed in a particular lumen, for example, the second lumen, inside the balloon blocking guide catheter 310, so that liquid/gas can be infused into the blood vessel on the to-be-ablated portion through a gap between the radio frequency ablation catheter 320 and the second lumen. Alternatively, the radio frequency ablation catheter 320 may be disposed in another lumen other than the first lumen and the second lumen inside the b 310. An electrode support 321 is disposed on a far end of the radio frequency ablation catheter 320, and at least two electrodes 322 are disposed on the electrode support 321. The electrode support 321 can expand and contract. When the electrode support 321 expands, some or all of the multiple electrodes 322 abut against the wall. The electrodes 322 may be connected, through a corresponding lead inside the radio frequency ablation catheter, to the radio frequency generator disposed inside a radio frequency ablation instrument. At least one of the multiple electrodes 322 is a radio frequency electrode 325, and the radio frequency electrode 325 is connected to an output terminal of the radio frequency generator. At least one electrode is a loop electrode 326, and the loop electrode 326 is connected to a loop terminal of the radio frequency generator. Multiple radio frequency electrodes 325 may share one loop electrode 326 to form a loop. When the conductivity environment inside the blood vessel changes, the radio frequency ablation instrument controls different electrodes 322 on the far end of the radio frequency ablation catheter 320 to be conducted, so that a loop passing through a blood vessel wall can be formed between a corresponding radio frequency electrode and loop electrode, to conduct radio frequency. Therefore, in the radio frequency ablation device, no peripheral patch electrode needs to be used to form a loop.

One or more through-wall electrodes are further disposed on the far end of the radio frequency ablation catheter 320. The through-wall electrode may be disposed on the electrode support, or the through-wall electrode may be directly disposed on a strip-shaped connection catheter of the radio frequency ablation catheter. The through-wall electrode is hollow and communicates with a path inside the radio frequency ablation catheter, and is used to inject liquid/gas into the blood vessel wall. For example, when the electrode support is segment-shaped, the through-wall electrode may be disposed at an adherent location in the middle of the segment-shaped electrode support. For another example, when a shape of the electrode support is a strip-shaped puncture needle, the through-wall electrode may be directly disposed at a front segment of the strip-shaped puncture needle. When the through-wall electrode is disposed on the electrode support, the through-wall electrode may be independently disposed, and the through-wall electrode may also serve as a radio frequency electrode or a loop electrode.

The following describes the radio frequency ablation device provided in the present invention and a radio frequency ablation principle thereof with reference to FIG. 6 to FIG. 8.

### Embodiment 1

As shown in FIG. 6, the radio frequency ablation device includes a guide catheter 310 and a radio frequency ablation catheter 320, and further includes a radio frequency ablation instrument (not shown in the figure) used to control a radio frequency process. The radio frequency ablation catheter 320 is connected to the radio frequency ablation instrument. The radio frequency ablation catheter 320 includes a strip-shaped connection catheter, an electrode support 321 disposed on a far end of the connection catheter, and a control handle disposed on a near end of the connection catheter. During use, the control handle is connected to the radio frequency ablation instrument through a composite cable, and multiple leads used to connect different electrodes and a radio frequency generator are disposed in the composite cable. Multiple electrodes 322 are disposed on the electrode support 321. After a front end of the radio frequency ablation catheter 320 extends out of the guide catheter 310, and the multiple electrodes are adherent under the action of the control handle, the radio frequency instrument may separately control different electrodes 322 on the front end of the radio frequency ablation catheter 320, to release a radio frequency. After a conductivity environment inside the blood vessel on the to-be-ablated portion is changed, during the radio frequency releasing process, a loop can be formed by using a blood vessel wall between different electrodes to conduct radio frequency ablation. In the radio frequency ablation device, no peripheral patch electrode needs to be used.

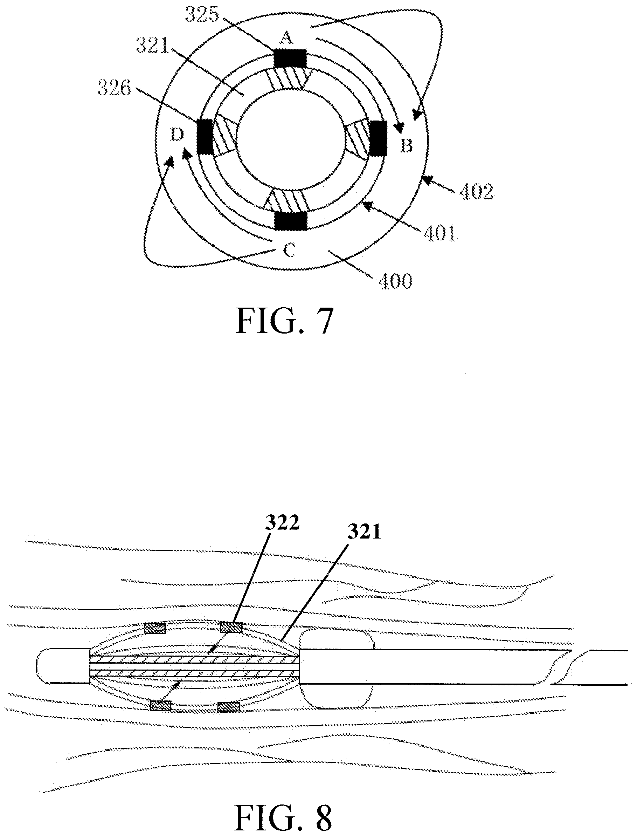

As shown in FIG. 6 and FIG. 7, after the inflated closed balloon 311 blocks the local blood flow in the blood vessel on the to-be-ablated portion, a contrast agent or other non-conductive liquid is infused into the lumen on the to-be-ablated portion through the second catheter branch 313, to control the radio frequency electrode to be adherent, and the radio frequency electrode 325 and the loop electrode 326 may form a loop through the blood vessel wall, to conduct radio frequency. In this case, as shown in FIG. 7, some radio frequency currents may enter into a blood vessel wall 400 through the radio frequency electrode 325 inside the lumen, and returns back to the loop electrode 326 inside the lumen after passing through the blood vessel wall 400 between an inner surface 401 and an outer surface 402 of the blood vessel wall. At the same time, some radio frequency currents may pass through the blood vessel wall 400 from the inner surface 401 of the blood vessel wall, pass through the outer surface 402 of the blood vessel wall, enters the blood vessel wall 400 from the outside of the outer surface 402 of the blood vessel wall, and then, returns to the loop electrode 326 through the blood vessel wall 400. During this process, a ratio of the some radio frequency currents flowing between the inner surface 401 and the outer surface 402 of the blood vessel wall to the some radio frequency currents passing through the blood vessel wall and entering the blood vessel wall depends on a difference between conductivity of the blood vessel wall and conductivity of a peripheral tissue of the blood vessel wall.

In this case, because the contrast agent is a poor conductor, and has high resistance, an environment inside the lumen is a non-conductive environment, and is in a non-conducted state. In this case, a peripheral electrode needs to be used, and mutual electron motion can be implemented between electrodes through the blood vessel wall, to implement radio frequency. A radio frequency emission direction between electrodes can be controlled by controlling a radio frequency sequence of the electrodes, to implement radio frequency ablation on nerve tissues on different ports of the outside of the blood vessel wall. When the foregoing solution is used, the blood vessel wall is a conductor, and because the blood vessel wall is close to the nerve, there is a small radio frequency loss, and the ideal radio frequency ablation effect of the nerve is achieved. Therefore, it is appropriate to infuse liquid used to reduce conductivity in the lumen, for example, non-conductive liquid, into the blood vessel in which blood flow is blocked, and good nerve ablation effect can be achieved. In addition, the non-conductive liquid infused into the lumen of the blood vessel can further reduce the temperature of the local lumen, and protect the local blood vessel. Certainly, gas may be alternatively infused into the blood vessel in which blood flow is blocked, to achieve a same objective.

As shown in FIG. 8, liquid or gas may be infused into the closed balloon 311 through the first catheter branch 312, to implement an inflation effect of the closed balloon 311, and block blood flow in the blood vessel. Normal saline may be infused into the blood vessel on the to-be-ablated portion through the second catheter branch 313. After the normal saline or other conductive liquid is infused into the blood vessel on the to-be-ablated portion through the second catheter branch 313, when the radio frequency electrode is adherent and emits a radio frequency current, because the normal saline is conductive, an environment in the lumen is a conductive environment, and communication is implemented in the lumen, electrons of the electrode freely moves inside the blood vessel, to form a loop inside the lumen. In some nerve ablation operations, normal saline needs to be infused into the blood vessel lumen. In this case, liquid/gas used to reduce local resistance of the blood vessel wall may be injected into the blood vessel wall, so that the blood vessel wall has good conductivity relative to the environment inside the lumen. Therefore, an ablation loop similar to that shown in FIG. 6 and FIG. 7 can be formed in the blood vessel wall and the peripheral tissue thereof, thereby achieving the nerve tissue ablation effect.

### Embodiment 2

In this embodiment, a structure of the radio frequency ablation device is basically the same as that in Embodiment 1, and includes the balloon blocking guide catheter, a radio frequency ablation catheter, and a radio frequency ablation instrument connected to the radio frequency ablation catheter. A difference between Embodiment 2 and Embodiment 1 lies in that a hollow through-wall electrode is further disposed on a far end of the radio frequency ablation catheter. The through-wall electrode is hollow and communicates with an internal path of the radio frequency ablation catheter, and is used to inject liquid used to change resistance of a blood vessel wall into the blood vessel wall. For example, normal saline used to reduce the resistance may be infused. The through-wall electrode may be a radio frequency electrode or a loop electrode disposed on an electrode support, or may be a separately disposed electrode dedicated for injection.

An outlet is disposed on a far end of the through-wall electrode, an inlet is disposed on a near end of the through-wall electrode, and the inlet communicates with the internal path of the radio frequency ablation catheter. An infusion tube communicating with the internal path is disposed on a rear end of the radio frequency ablation catheter, and the infusion tube is connected to an infusion device. The infusion device may inject, through the hollow through-wall electrode and the infusion tube, a material used to reduce local resistance of a blood vessel wall, for example, normal saline, into a wall tissue near a radio frequency ablation point, to increase a conductivity degree and a conductivity probability when the electrodes pass through the blood vessel wall, and reduce a degree and probability of conductivity between the electrodes inside the blood vessel lumen during radio frequency ablation.

Therefore, during the radio frequency ablation process, the closed balloon disposed on the far end of the guide catheter is inflated to block local blood flow inside the blood vessel, liquid/gas used to reduce conductivity inside the lumen is infused into the blood vessel through the guide catheter, and at the same time, liquid used to reduce local resistance is injected into the wall tissue of the blood vessel through the hollow through-wall electrode, and then, a loop may be formed between the radio frequency electrode and the loop electrode through the blood vessel wall by controlling different electrodes of the radio frequency ablation catheter, to conduct radio frequency ablation. Therefore, the nerve ablation effect is better than that in Embodiment 1.

Specifically, the through-wall electrode disposed on the far end of the radio frequency ablation catheter 2 may be disposed at an adherent location in the middle of a segment-shaped radio frequency electrode, or may be disposed at a front segment of a strip-shaped puncture needle radio frequency electrode. In addition, a front end of the through-wall electrode is a sharp acute angle and may have an edge, a shape of the through-wall electrode is a cone, a rhombus, or the like, a length range of the through-wall electrode is preferably 0.01 to 20 mm, and a diameter range of the through-wall electrode is preferably 0.01 to 2.0 mm.

### Embodiment 3

In this embodiment, to inject liquid used to reduce local resistance into a blood vessel wall on a to-be-ablated portion, a hollow puncture needle is disposed on a front end of the radio frequency ablation catheter, to replace an injection function of the hollow through-wall electrode in Embodiment 2. A through-wall electrode or a common electrode disposed in this embodiment only has a radio frequency ablation function, or may have a hollow structure and have an injection function. After the through-wall electrode penetrates or passes through the blood vessel wall, the through-wall electrode may directly release energy to a nerve plexus near the blood vessel wall, to reduce injury caused to the blood vessel wall during the radio frequency process. For introduction of the through-wall electrode, refer to the description made by the applicant in the prior patent application "CATHETER AND DEVICE FOR NERVE ABLATION IN CAVITY-PASSING AND WALL-PENETRATING MODE AND METHOD FOR NERVE ABLATION" (patent application number: CN201310049148.X). In this embodiment, the remaining structure of the radio frequency ablation device is basically the same as that in Embodiment 2, and includes the balloon blocking guide catheter, the radio frequency ablation catheter, and a radio frequency ablation instrument connected to the radio frequency ablation catheter.

A liquid cavity communicating with the puncture needle is disposed in the radio frequency ablation catheter provided in this embodiment, and is connected to an external infusion device through a catheter branch. The infusion device may inject, through the catheter branch and puncture needle, a material used to reduce local resistance of a blood vessel wall, for example, normal saline, into a wall tissue near a radio frequency ablation point, to increase a conductivity degree and a conductivity probability when the electrodes pass through the blood vessel wall, and reduce a degree and probability of conductivity between the electrodes inside the blood vessel lumen during radio frequency ablation.

Therefore, during the radio frequency ablation process, the closed balloon disposed on the far end of the guide catheter is inflated to block local blood flow inside the blood vessel, liquid/gas used to reduce conductivity inside the blood vessel is infused into the blood vessel through the guide catheter, and preferably, liquid/gas used to reduce conductivity is infused, to reduce a conductivity environment and a temperature environment inside the blood vessel on the to-be-ablated portion. At the same time, liquid used to reduce local resistance of the blood vessel wall is injected into a blood vessel wall tissue through the puncture needle. Then, a loop may be formed between the radio frequency electrode and the loop electrode through the blood vessel wall by controlling different electrodes of the radio frequency ablation catheter, to conduct radio frequency ablation. Therefore, the nerve ablation effect is better than that in Embodiment 1.

To sum up, in the radio frequency ablation device provided in the present invention and comprising the balloon blocking guide catheter, the closed balloon on the far end of the balloon blocking guide catheter is inflated, so that local blood flow inside the blood vessel on the to-be-ablated portion can be blocked. Liquid/gas used to change the conductivity environment or temperature environment is infused into the blood vessel on the to-be-ablated portion, and different electrodes of the radio frequency ablation catheter are controlled, so that a loop can be formed between the radio frequency electrode and the loop electrode through the blood vessel wall or the peripheral tissue thereof, to conduct radio frequency. In the radio frequency ablation device using the guide catheter, a radio frequency emission direction between electrodes can be controlled without a peripheral electrode, and the loop is formed between different electrodes through the blood vessel wall, to conduct radio frequency ablation. Compared with a loop passing through the entire human body, the loop formed between the radio frequency electrode and the loop electrode and passing through the blood vessel wall is a loop formed in a partial area, and needs to overcome small impedance of the human body. Because the blood vessel wall is close to the nerve, and no conductivity is implemented inside the chamber, a radio frequency loss is small, and an ideal nerve ablation effect is achieved.

In addition, liquid/gas used to reduce local resistance may be further infused into the blood vessel wall through the hollow through-wall electrode or the puncture needle, to increase a conductivity degree and a conductivity probability when the electrodes pass through the blood vessel wall, and reduce a degree and probability of conductivity between the electrodes inside the blood lumen during radio frequency ablation. In addition, liquid is injected into the blood vessel lumen and the blood vessel wall tissue, so that a local temperature can be reduced, and a local blood vessel can be protected.

The radio frequency ablation instrument in the present invention has a multichannel radio frequency output function, and a loop can be formed between multiple electrodes and through the blood vessel wall. The radio frequency ablation instrument loads radio frequency energy to a blood vessel, muscle, and nerve attached to the ablation catheter through various electrodes of the ablation catheter, and a radio frequency current sequentially passes through the blood vessel wall and the loop electrode, and returns to the radio frequency ablation instrument, to form a radio frequency loading loop. The radio frequency current generates high-speed ion vibration in the attached tissues, and generates a temperature rise, to achieve an ablation objective.

In the technical solution provided in the present invention, by using the foregoing radio frequency ablation instrument, a partial radio frequency current loop can be formed between two electrodes through the blood vessel wall, and the radio frequency energy is released only between two electrodes forming the loop, and a temperature is generated, thereby greatly reducing much radio frequency energy consumed by human body impedance.

A working principle of the radio frequency ablation instrument has been described in detail in the two prior patent applications: "RADIO FREQUENCY ABLATION METHOD AND RADIO FREQUENCY ABLATION SYSTEM FOR NERVE ABLATION" (patent application number: CN201410035836.5) and "RADIO FREQUENCY ELECTRODE WITH TEMPERATURE MEASUREMENT FUNCTION AND IMPEDANCE MEASUREMENT FUNCTION AND ABLATION INSTRUMENT" (patent application number: CN201310530007.X), and details are not described herein again.

The foregoing has described in detail the radio frequency ablation device comprising a balloon blocking guide catheter in the present invention.

## Claims

1. A radio frequency ablation device (300), wherein the radio frequency ablation device comprises a balloon blocking guide catheter (310), wherein the balloon blocking guide catheter comprises a dual-chamber or multi-chamber catheter, an inflatable closed balloon (311) configured to block local blood flow inside a blood vessel is disposed on an outer wall of a far end of the guide catheter, a first catheter branch (312) communicating with a first lumen inside the guide catheter is disposed on a near end of the guide catheter, the inside of the closed balloon is configured to communicate with the first lumen inside the guide catheter, the first catheter branch is configured to provide an inflation material for the closed balloon through the first lumen, a second catheter branch (313) communicating with a second lumen inside the guide catheter is disposed on the near end of the guide catheter, an infusion port is disposed on a far end of the second lumen, and the second catheter branch is configured to infuse liquid or gas into a blood vessel on a to-be-ablated portion through the infusion port; and
the radio frequency ablation device further comprises a radio frequency ablation catheter disposed in a particular lumen inside the balloon blocking guide catheter, wherein an electrode support (321) is disposed on a far end of the radio frequency ablation catheter, at least two electrodes (322) are disposed on the electrode support, at least one of the electrodes is connected to an output terminal of a radio frequency generator, to form a radio frequency electrode, and at least one of the electrodes is configured to be connected to a loop terminal of the radio frequency generator, to form a loop electrode; **characterized in that** one or more through-wall electrodes are disposed on the far end of the radio frequency ablation catheter, and the through-wall electrode is hollow and configured to communicate with a path inside the radio frequency ablation catheter, and is used to inject liquid or gas into a blood vessel wall.

2. The radio frequency ablation device according to claim 1, wherein
the electrode support is configured to expand and contract.

3. The radio frequency ablation device according to claim 1, wherein
the radio frequency ablation catheter is disposed in the second lumen inside the balloon blocking guide catheter.

4. The radio frequency ablation device according to claim 1, wherein
the through-wall electrode is disposed at an adherent location in the middle of a segment-shaped electrode support.

5. The radio frequency ablation device according to claim 1, wherein
the through-wall electrode is disposed at a front segment of a strip-shaped puncture needle.

## Patentansprüche

1. Funkfrequenzablationsvorrichtung (300), worin die Funkfrequenzablationsvorrichtung einen Ballonblockierungsführungskatheter (310) umfasst, worin der Ballonblockierungsführungskatheter einen Zweikammer- oder Mehrkammerkatheter umfasst, ein aufblasbarer geschlossener Ballon (311), der dafür ausgelegt ist, lokalen Blutfluss innerhalb eines Blutgefäßes zu blockieren, an einer Außenwand eines fernen Endes des Führungskatheters angeordnet ist, ein erster Katheterzweig (312), der mit einem ersten Lumen innerhalb des Führungskatheters kommuniziert, an einem nahen Ende des Führungskatheters angeordnet ist, das Innere des geschlossenen Ballons dafür ausgelegt ist, mit dem ersten Lumen innerhalb des Führungskatheters zu kommunizieren, der erste Katheterzweig dafür ausgelegt ist, ein Aufblasmaterial für den geschlossenen Ballon durch das erste Lumen bereitzustellen, ein zweiter Katheterzweig (313), der mit einem zweiten Lumen innerhalb des Führungskatheters kommuniziert, am nahen Ende des Führungskatheters angeordnet ist, ein Infusionsanschluss an einem fernen Ende des zweiten Lumens angeordnet ist, und der zweite Katheterzweig dafür ausgelegt ist, Flüssigkeit oder Gas in ein Blutgefäß an einem zu abladierenden Abschnitt durch den Infusionsanschluss zu infundieren; und
die Funkfrequenzablationsvorrichtung ferner einen Funkfrequenzablationskatheter umfasst, der in einem besonderen Lumen innerhalb des Ballonblockierungsführungskatheters angeordnet ist, worin ein Elektrodenträger (321) an einem fernen Ende des Funkfrequenzablationskatheters angeordnet ist, zumindest zwei Elektroden (322) am Elektrodenträger angeordnet sind, zumindest eine der Elektroden mit einer Ausgangsklemme eines Funkfrequenzgenerators verbunden ist, um eine Funkfrequenzelektrode zu bilden, und zumindest eine der Elektroden zu bilden, und zumindest eine der Elektroden dafür ausgelegt ist, mit einer Schleifenklemme des Funkfrequenzgenerators verbunden zu werden, um eine Schleifenelektrode zu bilden; **dadurch gekennzeichnet, dass** eine oder mehrere durch die Wand geführte Elektroden am fernen Ende des Funkfrequenzablationskatheters angeordnet sind, und die durch die Wand geführte Elektrode hohl ist und dafür ausgelegt ist, mit einem Pfad innerhalb des Funkfrequenzablationskatheters zu kommunizieren, und verwendet wird, um Flüssigkeit oder Gas in eine Blutgefäßwand zu injizieren.

2. Funkfrequenzablationsvorrichtung nach Anspruch 1, worin der Elektrodenträger dafür ausgelegt ist, sich auszudehnen und zu schrumpfen.

3. Funkfrequenzablationsvorrichtung nach Anspruch 1, worin der Funkfrequenzablationskatheter im zweiten Lumen innerhalb des Ballonblockierungsführungskatheters angeordnet ist.

4. Funkfrequenzablationsvorrichtung nach Anspruch 1, worin die durch die Wand geführte Elektrode an einer anliegenden Stelle in der Mitte eines segmentförmigen Elektrodenträgers angeordnet ist.

5. Funkfrequenzablationsvorrichtung nach Anspruch 1, worin die durch die Wand geführte Elektrode an einem vorderen Segment einer streifenförmigen Punktionsnadel angeordnet ist.

## Revendications

1. Dispositif d'ablation par radiofréquence (300),
dans lequel le dispositif d'ablation par radiofréquence comprend un cathéter de guidage de blocage à ballonnet (310),
dans lequel le cathéter de guidage de blocage à ballonnet comprend un cathéter à deux chambres ou à plusieurs chambres, un ballonnet fermé gonflable (311) configuré pour bloquer la circulation sanguine locale à l'intérieur d'un vaisseau sanguin est disposé sur une paroi extérieure d'une extrémité éloignée du cathéter de guidage, une première branche de cathéter (312) communiquant avec une première
lumière à l'intérieur du cathéter de guidage est disposée sur une extrémité proche du cathéter de guidage, la partie interne du ballonnet fermé étant configurée pour communiquer avec la première lumière à l'intérieur du cathéter de guidage,
la première branche de cathéter est configurée pour fournir un matériau de gonflage pour le ballonnet fermé à travers la première lumière, une seconde branche de cathéter (313) communiquant avec une seconde lumière à l'intérieur du cathéter de guidage est disposée sur l'extrémité proche du cathéter de guidage, un orifice de perfusion est disposé sur une extrémité éloignée de la seconde lumière, et la seconde branche de cathéter est configurée pour injecter un liquide ou un gaz dans un vaisseau sanguin sur une partie à ablater à travers l'orifice de perfusion ; et
le dispositif d'ablation par radiofréquence comprend en outre un cathéter d'ablation par radiofréquence disposé dans une lumière particulière à l'intérieur du cathéter de guidage de blocage à ballonnet, dans lequel un support d'électrode (321) est disposé sur une extrémité éloignée du cathéter d'ablation par radiofréquence, au moins deux électrodes (322) sont disposées sur le support d'électrode, au moins l'une des électrodes est reliée à un terminal de sortie d'un générateur de radiofréquence, pour former une électrode de radiofréquence, et au moins l'une des électrodes est configurée pour être reliée à un terminal de circuit du générateur de radiofréquence, pour former une électrode de circuit ; **caractérisé en ce que**
une ou plusieurs électrodes traversantes sont disposées sur l'extrémité éloignée du cathéter d'ablation par radiofréquence, et l'électrode traversante est creuse et configurée pour communiquer avec un parcours à l'intérieur du cathéter d'ablation par radiofréquence, et est utilisée pour injecter un liquide ou un gaz dans une paroi de vaisseau sanguin.

2. Dispositif d'ablation par radiofréquence selon la revendication 1, dans lequel le support d'électrode est configuré pour se dilater et se contracter.

3. Dispositif d'ablation par radiofréquence selon la revendication 1, dans lequel le cathéter d'ablation par radiofréquence est disposé dans la seconde lumière à l'intérieur du cathéter de guidage de blocage à ballonnet.

4. Dispositif d'ablation par radiofréquence selon la revendication 1, dans lequel l'électrode traversante est disposée au niveau d'un emplacement adhérent au milieu d'un support d'électrode en forme de segment.

5. Dispositif d'ablation par radiofréquence selon la revendication 1, dans lequel l'électrode traversante est disposée au niveau d'un segment avant d'une aiguille de ponction en forme de bande.
